Europäisches Patentamt

European Patent Office

Office européen des brevets

(11) Publication number: **0 091 881**
**A1**

# EUROPEAN PATENT APPLICATION

(21) Application number: **83730037.5**

(22) Date of filing: **08.04.83**

(51) Int. Cl.³: **C 07 J 53/00**
**A 61 K 31/585**

(30) Priority: **13.04.82 DE 3213962**

(43) Date of publication of application:
**19.10.83 Bulletin 83/42**

(84) Designated Contracting States:
**AT BE CH DE FR GB IT LI LU NL SE**

(71) Applicant: SCHERING AKTIENGESELLSCHAFT Berlin
und Bergkamen
Müllerstrasse 170/178 Postfach 65 03 11
D-1000 Berlin 65(DE)

(72) Inventor: Laurent, Henry, Dr.
Glambecker Weg 21
D-1000 Berlin 28(DE)

(72) Inventor: Bittler, Dieter
Bölkauer Pfad 11
D-1000 Berlin 27(DE)

(72) Inventor: Wiechert, Rudolf, Prof. Dr.
Petzowerstrasse 8A
D-1000 Berlin 39(DE)

(72) Inventor: Losert, Wolfgang, Prof. Dr.
Landshuter Strasse 22
D-1000 Berlin 30(DE)

(72) Inventor: Schillinger, Ekkehard, Dr.
im Amseltal 50
D-1000 Berlin 28(DE)

(54) 1-Alpha,2-alpha;6-beta,7-beta;15-beta,16-beta-Trimethylene-3-oxo-17-alpha-pregn-4-ene-21,17-carbolactone, its preparation and use as a medicinal agent.

(57) 1α,2α;6β,7β;15β,16β-trimethylene-3-oxo-17α-pregn-4-ene-21,17-carbolactone is prepared from 6β,7β;15β,16β-dimethylene-3-oxo-17α-pregna-1,4-diene-21,17-carbolactone with dimethylsulfoxonium methylide, and is used as a medicinal agent with aldosterone-antagonistic activity.

EP 0 091 881 A1

## 1α,2α;6β,7β;15β,16β-TRIMETHYLENE-3-OXO-17α-PREGN-4-ENE-21,17-CARBOLACTONE, ITS PREPARATION AND USE AS A MEDICINAL AGENT

The present invention relates to a new pharmacological agent.

### Summary of the Invention

It is an object of this invention to provide a new compound having valuable pharmacological properties.

Upon further study of the specification and appended claims, further objects and advantages of this invention will become apparent to those skilled in the art.

These objects have been achieved by providing 1α,2α; 6β,7β;15β,16β-trimethylene-3-oxo-17α-pregn-4-ene-21,17-carbolactone.

### Detailed Discussion

The invention furthermore concerns a process for the preparation of 1α,2α;6β,7β;15β,16β-trimethylene-3-oxo-17α-pregn-4-ene-21,17-carbolactone, comprising conventionally introducing a methylene group in the 1,2-position of the corresponding 6β,7β;15β,16β-dimethylene-3-oxo-17α-pregna-1,4-diene-21,17-carbolactone. The introduction of the methylene group in the 1,2-position of 6β,7β;-15β,16β-di-methylene-3-oxo-17α-pregna-1,4-diene-21,17-carbolactone can be accomplished in a manner known per se according to the process by Corey (E.J. Corey and M. Chaykovsky, J. Amer. Chem. Soc. 84 : 867 [1962]), whose disclosure is incorporated by reference herein. The starting material is known (U.S.P. 4,291,029).

For this purpose, dimethylsulfoxonium methylide is first prepared, e.g., from trimethylsulfoxonium iodide in dimethyl sulfoxide and sodium hydride in mineral oil under an inert gas atmosphere.

The starting steroid, dissolved in dimethyl sulfoxide, is then added to this reaction solution, and the mixture is reacted. The reaction is completed after 15-20 hours.

The reaction mixture is then worked up as usual, e.g., by precipitation, extraction, recrystallization, and/ or chromatography.

The compound of this invention possesses valuable pharmacological properties. It is, inter alia, a diuretic of the aldosterone antagonist type, i.e., it reverses the effect of deoxycorticosterone on the excretion of sodium and potassium. The compound of this invention surprisingly proves to be 3.5 times more effective than the known spironolactone in a test model by Hollmann (G. Hollmann et al., "Tubulaere Wirkungen und renale Elimination von Spirolactonen" [Tubular Effects and Renal Elimination of Spirolactones], Naunyn-Schmiedebergs Arch. Exp. Path. Pharmak. 247 : 419 [1964]; P. Marx, "Renale Wirkungen des d-Aldosterons und seines Antagonisten Spironolacton" [Renal Effects of d-Aldosterone and Its Antagonist Spironolactone], Diss. Med. Fak. FU Berlin 1966).

The compound of this invention is utilized by means of conventional methods of galenic pharmacy for the preparation of medicines for oral and parenteral administration, e.g., to mammals including humans, oral administration being preferred.

In the case of human patients, the dosage of the compound of this invention is usually 5-100 mg/day. Suitable dosage forms include tablets, dragees, capsules, and similar forms containing, e.g., 5-50 mg of active ingredient. Administration is analogous to that of the conventional diuretic spironolactone.

The medical specialties of this invention are prepared in the usual way by converting the active agent into the desired forms of application with suitable additives, e.g., solutions, lotions, ointments, creams, or plasters. In the thus-formulated medicines, the concentration of active compound is dependent on the type of application.

Conventional excipients are pharmaceutically acceptable organic or inorganic carrier substances suitable for parenteral, enteral or topical application which do not deleteriously react with the active compounds. Suitable pharmaceutically acceptable carriers include but are not limited to water, salt solutions, alcohols, gum arabic, vegetable oils, polyethylene glycols, gelatine, lactose, amylose, magnesium stearate, talc, silicic acid, viscous paraffin, perfume oil, fatty acid monoglycerides and diglycerides, pentaerythritol fatty acid esters, hydroxymethylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds.

For parenteral application, particularly suitable are injectable sterile solutions, preferably oily or aqueous solutions, as well as suspensions, emulsions, or implants, including suppositories. Ampoules are convenient unit dosages.

For enteral application, particularly suitable are tablets, dragees, suppositories or capsules having talc

4          **0091881**

and/or a carbohydrate carrier of binder or the like, the carrier preferably being lactose and/or corn starch and/or potato starch. A syrup, elixir or the like can be used wherein a sweetened vehicle is employed. Sustained release compositions can be formulated including those wherein the active compound is protected with differentially degradable coatings, e.g., by microencapsulation, multiple coatings, etc.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following example, all temperatures are set forth uncorrected in degrees Celsius; unless otherwise indicated, all parts and percentages are by weight.

EXAMPLE 1

A solution of 2.2 g of trimethylsulfoxonium iodide in 35 ml of dimethyl sulfoxide is combined with 393 mg of sodium hydride (in the form of a 55% suspension in oil) and stirred for one hour under an argon atmosphere. Then, 729 mg of 6β,7β;15β,16β-dimethylene-3-oxo-17α-pregna-1,4-diene-21,17-carbolactone is added thereto, agitation is continued under these conditions for 20 hours, the reaction product is subsequently precipitated with sulfuric-acid-containing ice water; the precipitate is filtered off and dissolved in dichloromethane. After drying and evaporation, the residue is dissolved in 8.5 ml of tetrahydrofuran and 8.5 ml of methanol, the solution is combined with 0.85 ml of 8% sulfuric acid (vol-%), stirred for 10 minutes, and thereafter diluted with diethyl ether. The organic phase is subsequently washed neutral with water, dried, and evaporated. The residue is recrystallized from acetone/diisopropyl ether, yielding 540 mg of 1α,2α;6β,7β;15β,16β-trimethylene-3-oxo-17α-pregn-4-ene-21,17-carbolactone, mp 261.7°C.

$$[\alpha]_D^{23} = +11^O \text{ (chloroform)}.$$

UV: $\varepsilon_{258} = 15,500$ (methanol).

EXAMPLE 2

10.0 mg. of $1\alpha,2\alpha;6\beta,7\beta;15,16\beta$-trimethylene-3-oxo-17$\alpha$-pregn-4-ene-21,17-carbolactone are micronised (finely ground) and homogeneously mixed with:

80.0 mg. lactose (DAB (German Pharmacopoeia 7); USP XVII),

29.6 mg. microcristallin cellulose, and

0.4 mg. magnesium stearate (USP XVII), and compressed into tablets without previous granulation, these tablets having a weight of 120 mg., with a diameter of about 7 mm. and a thickness of 2.7-2.9 mm.

The preceding examples can be repeated with similar success by substituting the generically or specifically described reactants and/or operating conditions of this invention for those used in the preceding example.

From the foregoing description, one skilled in the art can easily ascertain the essential characteristics of this invention, and without departing from the spirit and scope thereof, can make various changes and modifications of the invention to adapt it to various usages and conditions.

WHAT IS CLAIMED IS:

1. 1α,2α;6β,7β;15β,16β-trimethylene-3-oxo-17α-pregn-4-ene-21,17-carbolactone.

2. A pharmacological composition having aldosterone-antagonistic activity comprising an effective amount of 1α,2α;6β,7β;15β,16β-trimethylene-3-oxo-17α-pregn-4-ene-21,17-carbolactone and a pharmacologically acceptable carrier.

3. A composition of claim 2 wherein the amount of the carbolactone is 5-50 mg.

4. A method of achieving a diuretic effect in a patient in need of such treatment comprising administering to the patient an effective amount of 1α,2α;6β,7β;15β,16β-trimethylene-3-oxo-17α-pregn-4-ene-21,17-carbolactone.

5. A method of claim 4 wherein the amount of carbolactone administered is 5-100 mg/day.

6. A method of claim 5 wherein the administration is oral.

## EUROPEAN SEARCH REPORT

Application number

EP 83 73 0037

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl. ³) |
| Y | EP-A-0 019 690 (SCHERING AG)<br>* Claims * | 1-6 | C 07 J 53/00<br>A 61 K 31/585 |
| Y | FR-A-2 370 755 (SCHERING AG)<br>* Page 1, claims * | 1-6 | |
| Y | DE-A-1 914 507 (MERCK CO.)<br>* Claims 1-5,7,13,19; page 1 * | 1-6 | |
| Y | US-A-3 753 979 (GLEN E. ARTH)<br>* Claims 1,3; column 2 * | 1-6 | |
| | | | **TECHNICAL FIELDS SEARCHED (Int. Cl. ³)**<br><br>C 07 J 53/00 |

The present search report has been drawn up for all claims

| Place of search | Date of completion of the search | Examiner |
|---|---|---|
| THE HAGUE | 19-07-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82